# EUROPEAN PATENT SPECIFICATION

(11) **EP 1 692 293 B1**
(45) Date of publication and mention of the grant of the patent: **10.03.2010**
(21) Application number: 04812550.4
(22) Date of filing: 01.12.2004
(51) Int. Cl.: C12N 15/861

(54) **ADENOVIRUS-TRANSFECTED PRIMARY CELLS AND METHODS OF PATHWAY MAPPING**
MIT ADENOVIRUS TRANSFIZIERTE PRIMÄRZELLEN SOWIE VERFAHREN ZUR WEGKARTIERUNG
CELLULES PRIMAIRES ADENOVIRUS TRANSFECTEES ET PROCEDES DE CARTOGRAPHIE DE VOIES

(30) Priority: 01.12.2003 US 526009 P
(43) Date of publication of application: 23.08.2006
(73) Proprietor: Aventis Pharmaceuticals Inc., Bridgewater, New Jersey 08807 (US)
(72) Inventor: HAHN, Chang, S., Princeton, NJ 08540 (US); LI, Li, Belle Mead, NJ 08502 (US)
(74) Representative: Bouvet, Philippe
(86) International application number: PCT/US2004/040054
(87) International publication number: WO 2005/054485

(56) References cited:
- HORWOOD NICOLE J ET AL: "High-efficiency gene transfer into nontransformed cells: utility for studying gene regulation and analysis of potential therapeutic targets." 2002, ARTHRITIS RESEARCH. 2002, VOL. 4 SUPPL 3, PAGE(S) S215 - S225 , XP002327569 ISSN: 1465-9905 abstract page 216, left-hand column page 218 - page 219
- POPE RICHARD ET AL: "Regulation of TNF-alpha expression in normal macrophages: The role of C/EBPbeta" August 2000 (2000-08), CYTOKINE, VOL. 12, NR. 8, PAGE(S) 1171-1181 , XP002327567 ISSN: 1043-4666 abstract
- WAN YISONG Y ET AL: "The survival of antigen-stimulated T cells requires NFkappaB-mediated inhibition of p73 expression." March 2003 (2003-03), IMMUNITY, VOL. 18, NR. 3, PAGE(S) 331-342 , XP002327568 ISSN: 1074-7613 page 332; figures 1,2

## Description

### BACKGROUND OF THE INVENTION.

### Field of the Invention

The invention also relates to the field of pathway mapping, and in particular to pathway mapping immunocytes that are not immortal cell lines. The invention further relates to the field of modulating the expression of intracellular signaling pathways.

### Description of Related Art

An intracellular signaling pathway is a set of genes whose expression is controlled by a cascade of gene-to-gene interactions begun by an initial stimulus. In other words, the initial stimulus of an intracellular signaling pathway controls the expression of each gene on the pathway either directly or indirectly through another gene on the pathway. Known signaling pathways are involved in a wide range of biological activity including immune response, development, mitosis, and inflammation, and include, but are not limited to, the mitogen-activated protein kinase ("MAPK") pathways, the protein kinase A ("PKA") signaling pathway, the protein kinase C ("PKC") signaling pathway, the Type I interferon signaling pathway, the calcineurin ("CaN") signaling pathway, the cis-acting nuclear factor of κB cells ("NFκB") signaling pathway, and the IκB kinase ("IKK") signaling pathway. Although defects in signaling pathways are implicated in a wide range of disease states, in many cases little is understood about a signaling pathway between the initial stimulus and the ultimate cellular response.

Pathway mapping refers generally to elucidating the steps between a pathway's initial stimulus and ultimate responses. For the purposes of this invention, "pathway mapping" refers to the determination of whether a candidate cis-acting regulatory element is controlled, either directly or indirectly, by a given signaling pathway. A known method of pathway mapping employs host cells from a cultured cell line that are transfected with a DNA plasmid containing a reporter gene operatively linked to a regulatory element to be tested. The use of reporter genes is well established in the art. Kain, SR, and Ganguly, S, Overview of Genetic Reporter Systems, Unit 9.6 in Current Protocols in Molecular Biology, Ed. Ausubel, FM, et al., (Wiley and Sons, NY, 1995). Horwood et al. (Arthritis Res 2002, 4 (suppl 3):S215-S225), Pope et al. (Cytokine, 2000, 12, (8):1171-1181); and Wan and DeGregori (Immunity, 2003; 18, 331-342) disclose methods of pathway mapping using adenoviral vectors.

Transfection refers to the introduction of foreign DNA into a host cell, such as a cultured mammalian cell. Transfection has proved to be a powerful tool for analyzing the function, regulation, and interaction of genes and their respective gene products. Procedures for transfecting cells are well-established in the art. Chemical and physical methods of transfection include DEAEdextran (Fox RM, et al, Biochemistry Oct 4; 16(20):4470-7, PMID 911769 (1977)), calcium phosphate (Pear, W. S., Nolan, G. P., Scott, M. L., & Baltimore, D. Proc. Natl. Acad. Sci. USA 90, 8392 (1993)), liposome-mediated transfection (Zhang WW, et al., Biotechniques Nov; 15(5):868-72 (1993)), micro-injection (Colbere-Garapin F, and Garapin AC, Dev Biol Stand; 55:267-71, PMID: 6329857 (1983)), and electroporation (Neumann E and Kakorin S, Technol Cancer Res Treat. Oct; 1 (5):329-40(2002)). The use of recombinant adenovirus vectors for transfection is also well established in the art. Berkner, KL and Sharp, PA Nucleic Acids Res. 11:6003-6020 (1983); Berkner, KL and Sharp, PA BioTechniques 6:616-629 (1988); Bett, AJ, Haddara, W, Prevec, L and Graham, FL PNAS USA 91:8802-8806 (1994); Hitt, M, Addison, CL, and Graham, FL Adv. Pharmacol. 40:137-206 (1997). Recombinant adenoviruses have been used to transfect. *inter alia,* cardiac tissue, Ardehali, A. J. Thor. Cardiovas. Surg. 111:246-252 (1996), and lymphocytes, Leon, RP, Hedlund. T, Meech, SJ, Li, S, Schaack, J, Hunger, SP, Duke, RC, and DeGregori, J PNAS USA 95: 13159-13164 (1998). Human adenoviruses are double-stranded DNA viruses which enter cells by receptor-mediated endocytosis. These viruses have been considered well suited for gene transfer because they are easy to grow and manipulate and they exhibit a broad host range in vivo and in vitro. Adenoviruses are able to infect quiescent as well as replicating target cells and persist extrachromosomally, rather than integrating into the host genome. Recombinant adenoviruses can accommodate relatively large segments of foreign DNA (about 7kb), and have the advantage of a high titer virus production.

Known vectors for reporter constructs used for pathway mapping include the pCRE-d2EGFP plasmid, available commercially from BD Biosciences Clontech. This plasmid contains a cyclic-AMP response element ("CRE") operatively linked to a green fluorescent protein ("GFP") reporter gene, and can be used to measure activity on pathways such as the Jun N-terminal kinase ("JNK") signaling pathway, the p38 MAPK signaling pathway, and the protein kinase A ("PKA") signaling pathway. This plasmid uses the pUC origin of replication for propagation in E. coli, and a viral f1 origin for production of single stranded DNA. This plasmid transfects in eukaryotic cells at a much lower efficiency than adenovirus-based vectors, and are only efficient enough to be used on cultured eukaryotic cells lines and not primary eukaryotic cell cultures. Because known vectors for pathway mapping of immunocytes are not efficient enough to be practical for use on primary immunocytes, pathway mapping using methods of the prior art can currently only be performed on cultured cell lines. However, immortalized cultured cell lines tend to have a significantly different patterns of protein expression than their non-immortalized counterparts. It is desirable to be able to perform pathway mapping on primary host cells of higher organisms without unnecessarily disrupting their cells' normal pattern of expression. It is especially desirable to be able to perform pathway mapping on primary immunocytes without having to transform them into immortal cell lines.

### BRIEF SUMMARY OF THE INVENTION

In one aspect, the invention relates to a method for determining whether a stimulus known to modulate expression of a signaling pathway in an immunocyte is capable of modulating expression of a candidate cis-element having the steps of transfecting the immunocyte with a recombinant adenovirus having a reporter gene operatively linked to the candidate cis-element; measuring a base level of reporter gene activity; applying the stimulus to the immunocyte; and measuring reporter gene activity in response to said stimulus. In a further aspect, the stimulus is modulating expression of a regulatory protein. In yet a further aspect, the method further comprises co-transfection with an expression system for the regulatory protein. In an alternate aspect, the stimulus is introducing a candidate regulatory compound. In a further aspect, the reporter gene is luciferase, green fluorescent protein ("GFP"), β-galactosidase ("GAL"), chloramphenicol acetyltransferase ("CAT"), or a supressor gene such as IκBsd. In another aspect, the cis-element is related to inflammation. In another aspect, the cis-element is AP-1, CRE, ISRE, NFAT, NFκB, or SRE. In another aspect, the immunocyte is a macrophage, CD4⁺ T cell, or immature dendritic cell.

In one aspect, the invention relates to a method for inhibiting expression of a signaling pathway in an immunocyte having the steps of transfecting the immunocyte with a recombinant adenovirus containing a suppressor gene operatively linked to a cis-element belonging to the signaling pathway; and inducing expression of the suppressor gene. In a further aspect, the signaling pathway is the NFκB signaling pathway. In yet a further aspect, the suppressor gene is IκBsd. In a further aspect, the immunocyte is a macrophage, CD4+ T cell, or an immature dendritic cell.

### BRIEF DESCRIPTION OF THE DRAWINGS

FIGURE 1A depicts macrophages transfected with a recombinant adenovirus capable of expressing green fluorescent protein ("AdV:GFP").
FIGURE 1B depicts dendritic cells transfected with a recombinant adenovirus capable of expressing green fluorescent protein ("AdV:GFP").
FIGURE 1C depicts green fluorescent protein expression in T lymphocytes.
FIGURE 1D depicts green fluorescent protein expression in B lymphocytes.
FIGURE 2 depicts cell two separate cell cultures, one transfected by a recombinant adenovirus capable of expressing a superdominant mutant of IκB ("Adv:IKBsd") and the other transfected by a recombinant adenovirus capable of expressing green fluorescent protein ("AdV:GFP"), each depicted in phase contrast and propidium iodide staining.
FIGURE 3A is a schematic diagram depicting a superdominant mutant of IκB ("IBsd") blocking the NFκB signaling pathway.
FIGURE 3B is a chart depicting the effects of inhibition of NFκB activation on inflammatory cytokine expression during dendritic cell maturation.
FIGURE 4 is a schematic depicting a recombinant adenovirus-based promoter/reporter system.
FIGURE 5A is a chart depicting the activation of CRE cis-elements in CD4⁺T cells upon stimulation.
FIGURE 5B is a chart depicting the activation of AP-1 cis-elements in CD4⁺T cells upon stimulation.
FIGURE 5C is a chart depicting the activation of NFκB cis-elements in CD4⁺T cells upon stimulation.
FIGURE 5D is a chart depicting the activation of ISRE cis-elements in CD4⁺T cells upon stimulation.
FIGURE 6A is a chart depicting the activation of cis-elements in macrophages.
FIGURE 6B is a chart depicting the activation of cis-elements in dendritic cells.
FIGURE 7A is a schematic depiction of the results of a hypothetical pathway mapping experiment.
FIGURE 7B is a schematic depiction of the results of a hypothetical pathway mapping experiment.

### DETAILED DESCRIPTION OF THE INVENTION

The present invention provides methods and compositions to determine the role of cis-acting regulatory elements in signaling pathways of primary immunocytes. For the purposes of this invention, "cis-acting regulatory element" is used interchangeably with "cis-element" and refers to a polynucleotide regulatory element operatively linked to a coding sequence wherein the regulatory element modulates the expression of the coding sequence. For the purposes of this invention, "intracellular signaling pathway" is used interchangeably with "signal transduction pathway," "signaling pathway," and "pathway," and refers to a collection of genes whose regulation is modulated, either directly or indirectly, by a given initial stimulus. In a typical signaling pathway, the initial stimulus activates a first cis-element which activates production of a corresponding first regulatory protein. The first regulatory protein then activates a second cis-element in the pathway, which in turn activates production of a second regulatory protein. The second regulatory protein then activates a third cis-element, and so on. In this example, the initial stimulus regulates the third cis-element indirectly. For the purposes of this invention a cis-element is identified as belonging on a pathway if it is regulated either directly or indirectly by the pathway's initial stimulus. For the purposes of this invention, "initial stimulus" refers to any phenomenon that modulates a pathway's activity. Initial stimuli include, but are not limited to, increased or decreased expression of a , regulatory protein, viral infection, contact with an allergen, DNA damage, and extracellular stress. Examples of extracellular stress include, but are not limited to, heat shock, osmotic stress, osmotic stress, pH stress, and ionizing radiation.

Many human diseases stem from one or more defects in the signaling pathway of immune cells such as macrophages, T-cells, and dendritic cells. For example, defects in the p38 mitogen activated protein kinase ("MAPK") pathway have been causally implicated in, *inter alia,* arthritis, sepsis, and human immunodeficiency virus infection. Similarly, defects in the Type I interferon signaling pathway result in an inadequate response to viral infection. Some known signaling pathways and their known component cis-elements are described below.

### Cis-Acting Regulatory Elements and their Pathways

A cis-acting regulatory element, also known as a cis-element, is a polynucleotide regulatory element operatively linked to a coding sequence wherein the regulatory element modulates the expression of the coding sequence. Examples of cis-acting regulatory elements include, but are not limited to, Activator Protein 1 ("AP-1"), cyclic-AMP response element ("CRE"), interferon stimulated response element ("ISRE"), nuclear factor activated T cells ("NFAT"), nuclear factor of κB cells ("NFκB"), and serum response element ("SRE").

The cis-acting Activator Protein 1 ("AP-1") enhancer element is modulated by the transcription factors c-jun and c-fos. The cis-acting AP-1 enhancer element appears in the c-jun N-terminal protein kinase ("JNK") signaling pathway. The JNK signaling pathway is a class of mitogen activated protein kinase ("MAPK") pathway, the MAPK signaling pathway has been implicated in the cellular response to several forms of extracellular stress, as well as cellular sensitivity to apoptosis.

The cis-acting cyclic-AMP response element ("CRE") enhancer element is modulated by the transcription factors ATF2 and CREB. The cis-acting CRE enhancer element appears in the JNK signaling pathway, the p38 signaling pathway, and the Protein Kinase A ("PKA") signaling pathway. Like the JNK signaling pathway, the p38 signaling pathway is a class of mitogen activated protein kinase ("MAPK") pathway and has been implicated in cellular response to several forms of extracellular stress and inflammatory cytokines. The PKA signaling pathway has been implicated in mitosis.

The cis-acting interferon stimulated response element ("ISRE") enhancer element is modulated by the transcription factors STAT1 and STAT2. The cis-acting ISRE enhancer element appears in the Type I interferon signaling pathway. The Type I interferon signaling pathway has been implicated in cellular response to viral infections.

The cis-acting nuclear factor activated T cells ("NFAT") enhancer element is modulated by the transcription factor NFAT. The cis-acting NFAT enhancer element appears in the protein kinase C ("PKC") signaling pathway as well as the calcineurin ("CaN") signaling pathway. The PKC signaling pathway has been implicated in action of histamine in vasoconstriction. The CaN signaling pathway has been implicated in hypertrophy of the heart.

The cis-acting nuclear factor of κB cells ("NFκB") enhancer element is modulated by the transcription factor NFκB. The cis-acting NFκB enhancer element appears in the IκB kinase ("IKK") signaling pathway as well as the NFκB signaling pathway. The IKK and NFκB signaling pathways have been implicated in the cellular response to several forms of extracellular stress.

The cis-acting serum response element ("SRE") enhancer element is modulated by the transcription factors Elk-1 and SRF. The cis-acting SRE enhancer element appears in the MAPK signaling pathway as well as the JNK signaling pathway.

The cis-elements listed above are known to belong to the respective pathway or pathways listed above. However, many cis-elements exist whose membership in signaling pathways is not determined. One embodiment of the invention provides a method for determining whether or not a candidate cis-element belongs to a given pathway. In this embodiment, a reporter construct containing the candidate cis-element operatively linked to a reporter gene is first transfected into a host primary cell such as an immunocyte. The host cell is then stimulated in a way that activates the pathway in question. Activity of the reporter gene is then measured. If the reporter gene activity is modulated in response to the stimulus, then the candidate cis-element is determined to belong on the pathway in question.

### Construction and Delivery of Reporter Construct

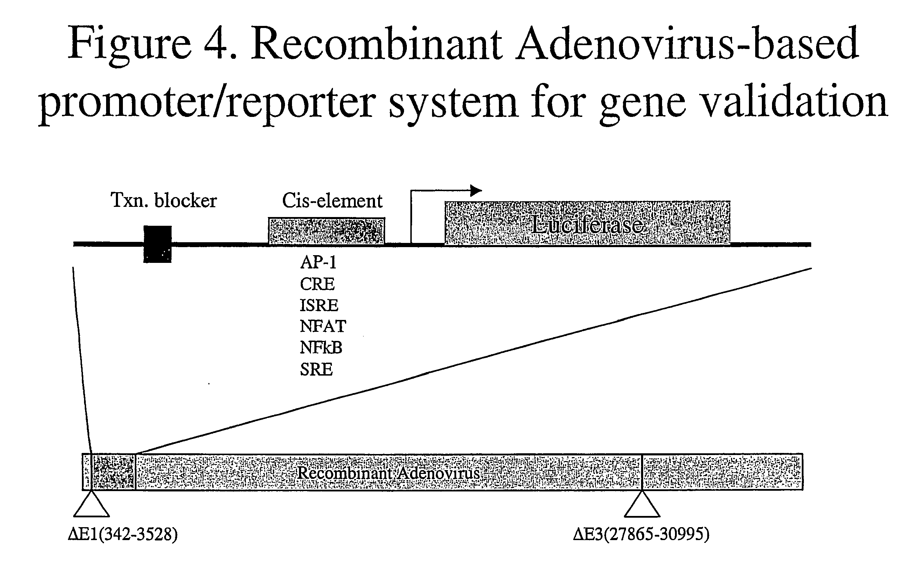

One embodiment of the invention uses a recombinant reporter construct comprising a reporter gene operatively linked to a candidate cis-element. In one embodiment, this recombinant reporter construct is delivered into a host cell by an adenovirus-based vector. FIGURE 4 is a schematic depicting a recombinant adenovirus-based vector. In one embodiment, this vector is derived from an adenovirus type 5 genome, such as the commercially available BD ADENO-X DNA, which is about 33 kb in length and has been altered by deleting large portions of the E1 and E3 region. The recombinant reporter insert comprises the candidate cis-element, a transcription blocker upstream of the candidate cis-element, and a reporter gene downstream of the candidate cis-element.

The upstream transcription blocker ("TB") element reduces background transcription levels of the reporter gene and may include one or more transcription pause elements or polyadenylation sites. Typical reporter genes include, but are not limited to, luciferase, β-galactosidase ("GAL"), chloramphenicol acetyltransferase ("CAT"), green fluorescent protein ("GFP"), and variants thereof, including but not limited to destabilized variants. In one embodiment, described in more detail below, the reporter gene is a superdominant mutant capable of blocking expression of a signaling pathway.

In one embodiment, the recombinant reporter insert is flanked by a I-Ceu I restriction site and a PI-Sce I restriction site, and ligated in vitro to the linearized BD ADENO-X DNA, which is pre-cut with I-Ceu I and PI-Sce I. The resulting DNA is grown in E. coli, and the purified plasmid is transfected into HEK293 cells. The recombinant viruses are rescued by transfection of the full-length recombinant adenovirus DNA into HEK293 cells and then amplified to generate high titer stocks.

### Determining if a Candidate Cis-Element Belongs in a Pathway

Once the reporter contstruct containing the candidate cis-element is cloned into the adenovirus-based vector, the reporter construct is then transfected into a host cell by bringing the vector into contact with the host cell at a sufficient multiplicity of infection ("MOI") and incubating for a sufficient amount of time. Examples of appropriate MOIs and incubation times are provided in the examples below.

Once the host cell is transfected with the reporter construct containing the candidate cis-element, a base level of reporter gene activity is measured. Then a stimulus known to activate the pathway in question is applied to the cell. For example, if the pathway in question is the p38 MAPK pathway, the stimulus applied could be an inflammatory cytokine, or cellular stress, such as heat shock or a hypertonic medium. After the stimulus is applied, activity of ther reporter gene is measured again and compared to its base level activity. If the reporter's activity is modulated by application of the stimulus, then the candidate cis-element is determined to belong to the pathway in question.

### EXAMPLES

The following examples are non-limiting. For example, the use of other cis-elements than those listed is contemplated, as is the use of other reporter and regulatory genes than those listed. For the purposes of this invention, "ex vivo" refers to the use of primary cell cultures in contrast to the use of immortalized cell lines.

### Example: Recombinant Adenovirus Infection in Primary Human Immunocytes

In this example, all major cell types of primary human immunocytes are examined for their susceptibility to adenoviral infection using a recombinant adenovirus capable of constitutively expressing green fluorescent protein ("AdV:GFP"). Such a recombinant adenovirus is available commercially from Quantum Biology. In this example, the types of primary human immunocytes successfully transfected by AdV:GFP include bone marrow macrophages, *is vitro* differentiated immature dendritic cells ("iDCs"), and peripheral blood mononuclear cells ("PBMCs"). The iDCs are prepared from CD14⁺ cells negatively selected from PBMCs by IL-4 and GM-CSF. The macrophages and the iDCs are infected with AdV:GFP at a multiplicity of infection ranging from 10 to 330 per cell, and then incubated for 16-24 hours. The cells are then examined under a fluorescent microscope for GFP expression.

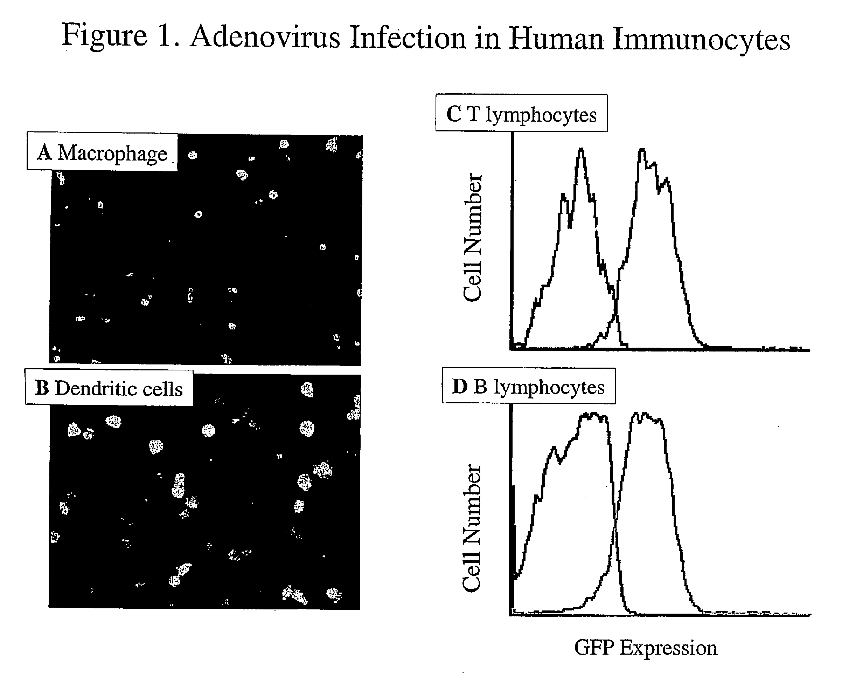

FIGURE 1A depicts GFP expression in transfected bone marrow macrophages and FIGURE 1B depicts GFP expression in transfected iDCs at 100 MOI. At this MOI, up to 90% of the cells show detectable expression of GFP.

Fresh peripheral blood mononuclear cells ("PBMCs") are infected with AdV:GFP at a MOI ranging from 100 to 1000 per cell. Twenty-four hours after infection, infected cells are labeled with monoclonal antibodies specific to CD4 and CD19.

FIGURE 1C depicts GFP expression in CD4⁺ T lymphocytes and FIGURE 1D depicts GFP expression in CD19⁺ B lymphocytes infected by AdV:GFP at 300 MOI. In both Figures, GFP expression is measured with FL1 set at 515nm using a FACSCalibur analyzer, available commercially from BD Biosciences. The shift in fluorescent intensity depicted in both of these figures represents a majority of cells in both cultures showing detectable expression of GFP.

### Example: Blocking the NFκB Signaling Pathway in Macrophages with AdV:IκBsd

In this example, a recombinant adenovirus capable of constitutively expressing a superdominant IκB mutant ("AdV:IKBsd") is constructed. The IκBsd mutant comprises two point mutations in human IκBa that changes serine 32 and serine 36 to alanines. Thus, the IκBsd mutant cannot be phosphorylated by IκB kinase-b. In this manner, the IκBsd mutant can block the NFκB signaling pathway by preventing proteasome-mediated degradation of the IκBa. In the absence of the NFκB signaling pathway, such as when it is blocked by the superdominant IκB mutant, TNF-α treatment induces apoptosis in various cell types including macrophages.

In this example, bone marrow macrophages are infected with either AdV:GFP as a control, or AdV:IκBsd. Both sets of macrophages are then treated with TNF-α at 10nM. Apoptosis is measured by propidium iodide ("PI") staining that stains DNA in the absence of cell permeabilization.

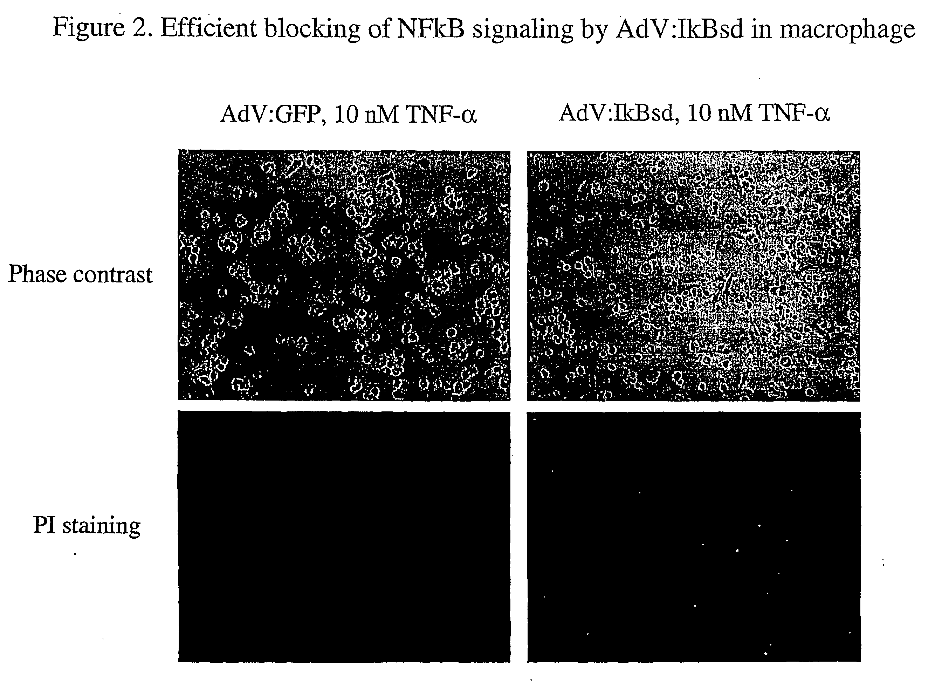

As illustrated in FIGURE 2, the control group AdV:GFP-whose NFκB signaling pathway remained intact-showed minimal induction of apoptosis, less than 5% of total cells. (Uninfected control and infected cells without TNF-α treatment also showed less than 5% of induction of apoptosis.) In contrast, the AdV:IκBsd cells exhibited induction of apoptosis in more than 75% of cells. Thus, AdV:IκBsd is shown to effectively block the NFκB signaling pathway in macrophages.

### Example: Blocking the NFκB Signaling Pathway in Dendritic Cells with AdV:IKBsd

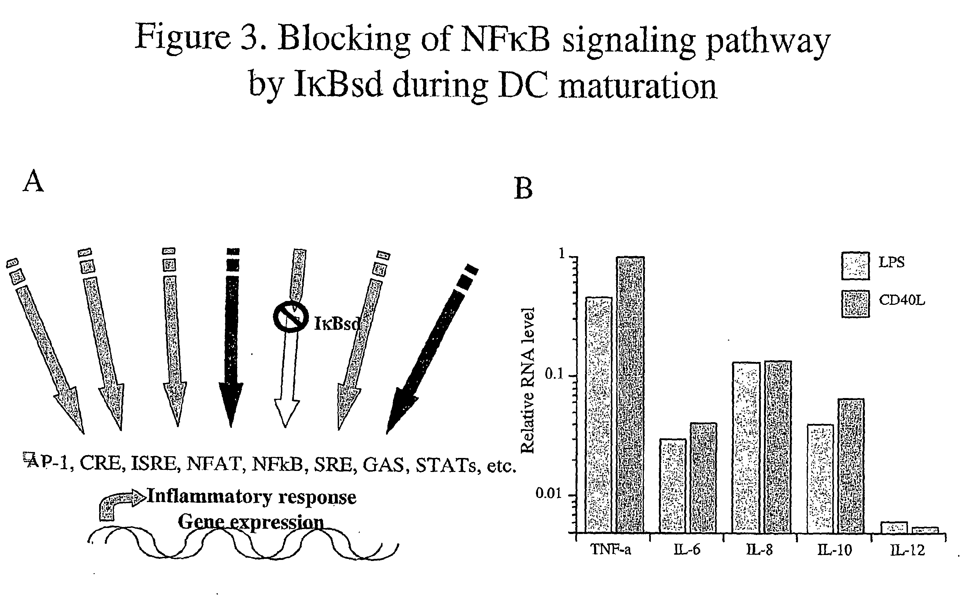

In this example, the effect of blocking the NFκB Signaling Pathway in *in vitro* differentiated human dendritic cells ("iDCs") is determined. FIGURE 3A is a schematic diagram depicting the signaling pathway of iDCs upon activation with either lipopolysaccharide ("LPS") or by CD40 ligand ("CD40L"), with the NFκB signaling pathway being blocked by IκBsd. LPS activates TLR4; CD40L activates CD40.

In this example, iDCs are obtained as follows: First, peripheral blood mononuclear cells ("PBMCs") are obtained by density gradient centrifugation. One density gradient centrifugation medium is FICOLL PAQUE, commercially available from Amersham Biosciences. Monocytes are then isolated from the PBMCs by magnetic depletion of non-monocytes. Finally, iDCs are isolated from the monocytes by treatment of the monocytes with IL4 and GM-GSF for 6 days.

In this example, resting cultures of isolated iDCs are then either (1) mock infected, (2) infected with AdV:GFP at a MOI of 100 as a control, or (3) infected with AdV:IKBsd at a MOI of 100. (For the purposes of this invention, "multiplicity of infection" ("MOI") refers to the ratio of infectious units to infected cells. Unless otherwise specified, multiplicity of infection is expressed as a ratio of infectious units to HEK293 T-cells.) After an incubation period of 16-24 hours, each set of iDCs is then either (1) mock activated, (2) activated by LPS at a concentration of 1 µg/ml or (3) activated by CD40L at a concentration of 1 µg/ml. The effects of inhibition of NFκB activation on inflammatory cytokine expression during iDC maturation was performed by real-time PCR analyses of selective cytokines and chemokines as well as a cytometric bead assay for various cytokine secretion. At 4-8 hours post activation, RNA is extracted and cytokine expression levels of various cytokines and chemokines are measured by TaqMan real-time PCR. Also, supernatants are collected at 8 hours post-activation and examined for cytokine secretion by cytometric bead assay.

FIGURE 3B is a chart depicting the expression of selected cytokines in AdV:IκBsd-infected cells relative to AdV:GFP-infected cells. In this example, AdV:IKBsd is shown to effectively block the NFκB signaling pathway in activated dendritic cells. Specifically, a significant reduction in IL-12, IL-10,1L-8, an IL-6 expression is shown in this figure.

### Example: Comparison of AdV:IκBsd to S0101627

In this example, a comprehensive study of inhibition of the NFkB signaling pathway during activation of dendritic cells by either LPS or CD40 ligand is performed using side-by side comparison of inhibition by either expression of super-dominant IkB or a known inhibitor compound of IkB kinase beta, S0101627. In this example, iDCs are either (1) mock infected, (2) infected with AdV:GFP as a control, or (3) infected with AdV:IkBsd for 12 hours followed by stimulation with either LPS or CD40L. Uninfected iDC is treated with S0101627 at the time of stimulation. 8 hours post stimulation, supernatants and cells are collected and further processed for cytometric bead assay for inflammatory cytokines and real time PCR for selective cytokines/chemokines expression, respectively. The real-time PCR data are shown in tables 1 and 2 for the expression of cytokines and chemokines, respectively.

**Table 1. Expression of selective cytokine expression upon inhibition of NFκB signaling pathway by chemical inhibitor (cmpd) or biochemically using adenovirus (AdV:IkB).**

| | | Control | StDev | Cmpd | StDev | AdV:GFP | StDev | AdV:lkB | StDev |
|---|---|---|---|---|---|---|---|---|---|
| **IL-1β** | cont | 29.263 | 3.529 | 34.064 | 2.985 | 14.642 | 0.112 | 13.813 | 2.783 |
| | LPS | 4602.917 | 116.731 | 185.004 | 15.267 | 10032.276 | 274.488 | 241.921 | 21.914 |
| | CD40L | 12262.184 | 269.214 | 422.791 | 10.747 | 16591.932 | 1384.342 | 422.584 | 18.477 |
| | | | | | | | | | |
| **IL-6** | cont | 6.931 | 0.549 | 9.073 | 1.482 | 60.976 | 1.680 | 18.473 | 2.771 |
| | LPS | 1448.169 | 110.883 | 10.393 | 5.527 | 4805.537 | 97.018 | 104.716 | 5.681 |
| | CD40L | 1538.403 | 55.857 | 4.601 | 1.040 | 6710.969 | 224.149 | 136.214 | 4.596 |
| | | | | | | | | | |
| **IL-8** | cont | 417.177 | 38.844 | 7008.142 | 306.451 | 895.411 | 28.116 | 949.533 | 15.990 |
| | LPS | 33310.859 | 669.480 | 29927.240 | 1011.946 | 59572.640 | 1490.781 | 6117.183 | 314.083 |
| | CD40L | 52413.686 | 710.084 | 26603.448 | 466.182 | 84912.576 | 832.407 | 9403.936 | 252.355 |
| | | | | | | | | | |
| **IL-10** | cont | 58.132 | 9.059 | 23.929 | 2.189 | 122.912 | 3.738 | 69.419 | 2.771 |
| | LPS | 131.546 | 10.705 | 27.550 | 5.941 | 1317.157 | 6.710 | 113.991 | 12.935 |
| | CD40L | 503.517 | 13.998 | 14.016 | 1.442 | 3213.362 | 29.764 | 210.082 | 2.872 |
| | | | | | | | | | |
| **IL-12** | cont | 0.489 | 0.159 | 0.035 | 0.000 | 0.166 | 0.293 | 0.067 | 0.000 |
| | LPS | 367.059 | 26.466 | 0.321 | 0.056 | 3275.257 | 113.158 | 33.137 | 6.168 |
| | CD40L | 3969.080 | 236.063 | 0.270 | 0.096 | 8956.589 | 512.201 | 239.002 | 14.327 |
| | | | | | | | | | |
| **TNF-α** | cont | 96.467 | 2.508 | 277.594 | 2.824 | 327.274 | 7.669 | 105.575 | 8.647 |
| | LPS | 2085.233 | 58.702 | 508.459 | 12.790 | 6372.957 | 5.395 | 1345.425 | 65.059 |
| | CD40L | 892.387 | 6.614 | 244.186 | 13.417 | 7137.164 | 155.537 | 2802.769 | 151.089 |

**Table 2. Expression of selective chemokine expression upon inhibition of NFκB signaling pathway by chemical inhibitor (cmpd) or biochemically using adenovirus (AdV:IkB).**

| | | Control | StDev | Cmpd | StDev | AdV:GFP | StDev | AdV:IkB | StDev |
|---|---|---|---|---|---|---|---|---|---|
| **CCL4** | Cont. | 78.24 | 9.65 | 391.81 | 33.49 | 344.23 | 15.58 | 81.04 | 2.70 |
| | LPS | 5506.79 | 207.13 | 731.64 | 19.09 | 16431.08 | 545.57 | 1052.61 | 115.21 |
| | CD40L | 2052.99 | 111.58 | 795.14 | 83.12 | 11834.94 | 136.51 | 3497.32 | 12.78 |
| | | | | | | | | | |
| **EXODUS-1** | Cont. | 0.05 | 0.00 | 0.00 | 0.00 | 0.16 | 0.01 | 0.20 | 0.00 |
| | LPS | 21.33 | 5.07 | 0.23 | 0.00 | 64.09 | 4.58 | 1.15 | 0.39 |
| | CD40L | 98.24 | 21.26 | 0.21 | 0.04 | 185.96 | 30.25 | 10.30 | 1.05 |
| | | | | | | | | | |
| **GRO-1** | Cont. | 0.59 | 0.29 | 2.75 | 0.98 | 1.87 | 0.12 | 0.98 | 0.20 |
| | LPS | 106.76 | 23.26 | 5.77 | 0.50 | 296.28 | 28.22 | 7.88 | 4.48 |
| | CD40L | 225.25 | 44.53 | 3.68 | 1.43 | 353.45 | 75.34 | 19.20 | 1.88 |
| | | | | | | | | | |
| **MIG** | Cont. | 0.45 | 0.19 | 0.38 | 0.21 | 17.79 | 1.57 | 0.55 | 0.33 |
| | LPS | 447.49 | 37.46 | 0.76 | 0.42 | 925.99 | 36.47 | 20.45 | 2.49 |
| | CD40L | 3.58 | 0.37 | 0.27 | 0.19 | 911.60 | 87.27 | 9.50 | 1.50 |
| | | | | | | | | | |
| **SCYA3** | Cont. | 88.52 | 8.01 | 284.19 | 18.69 | 417.14 | 77.22 | 167.58 | 10.86 |
| | LPS | 2578.07 | 358.01 | 1590.39 | 406.06 | 6361.56 | 681.21 | 1297.35 | 157.74 |
| | CD40L | 1055.66 | 74.36 | 1311.47 | 258.38 | 6604.14 | 1174.11 | 2853.10 | 94.21 |
| | | | | | | | | | |
| **TARC** | Cont. | 655.20 | 90.76 | 502.24 | 236.85 | 718.52 | 194.34 | 631.01 | 81.71 |
| | LPS | 1602.37 | 343.61 | 678.33 | 104.44 | 1337.75 | 140.59 | 602.76 | 63.24 |
| | CD40L | 4303.55 | 637.38 | 440.39 | 70.93 | 1448.82 | 225.74 | 624.01 | 63.94 |
| | | | | | | | | | |
| **SCYA19** | Cont. | 0.32 | 0.13 | 0.46 | 0.35 | 2.76 | 0.51 | 0.72 | 0.39 |
| | LPS | 298.64 | 11.87 | 0.21 | 0.08 | 1207.98 | 74.16 | 2.22 | 0.34 |
| | CD40L | 119.31 | 6.81 | 0.27 | 0.15 | 1682.39 | 44.62 | 2.22 | 0.22 |
| | | | | | | | | | |
| **RANTES** | Cont. | 14.49 | 2.70 | 13.89 | 1.29 | 401.75 | 57.96 | 18.88 | 1.97 |
| | LPS | 7512.60 | 4992.80 | 18.05 | 4.18 | 16745.39 | 2834.61 | 279.75 | 18.42 |
| | CD40L | 1274.12 | 283.33 | 16.41 | 1.56 | 14526.19 | 5679.46 | 75.28 | 7.54 |
| | | | | | | | | | |
| **SCYB11** | Cont. | 0.66 | 0.00 | 0.33 | 0.00 | 1029.52 | 76.69 | 1.08 | 0.46 |
| | LPS | 2343.81 | 382.47 | 1.30 | 0.23 | 8467.22 | 1551.04 | 224.25 | 52.98 |
| | CD40L | 11.73 | 2.70 | 0.45 | 0.29 | 5928.08 | 737.52 | 86.70 | 11.39 |
| | | | | | | | | | |
| **SCY1** | Cont. | 0.32 | 0.24 | 0.12 | 0.12 | 0.60 | 0.11 | 0.18 | 0.00 |
| | LPS | 132.21 | 33.22 | 0.63 | 0.34 | 164.18 | 11.05 | 24.59 | 2.33 |
| | CD40L | 28.39 | 1.80 | 0.39 | 0.24 | 51.26 | 3.57 | 22.25 | 5.19 |
| | | | | | | | | | |
| **SCYA10** | Cont. | 4.56 | 1.57 | 5.40 | 0.56 | 10.16 | 2.55 | 3.55 | 0.94 |
| | LPS | 7.72 | 1.34 | 6.49 | 0.14 | 8.43 | 2.69 | 5.29 | 1.15 |
| | CD40L | 1.81 | 0.15 | 1.95 | 0.42 | 12.10 | 0.92 | 3.43 | 1.51 |
| | | | | | | | | | |
| **SCYA22** | Cont. | 942.96 | 65.13 | 518.31 | 12.32 | 861.27 | 34.95 | 974.96 | 26.85 |
| | LPS | 3362.80 | 116.46 | 658.42 | 33.05 | 5233.39 | 280.99 | 1169.51 | 82.86 |
| | CD40L | 7862.22 | 135.85 | 527.75 | 42.69 | 8413.07 | 261.49 | 886.01 | 59.45 |
| | | | | | | | | | |
| **GRO2** | Cont. | 0.83 | 0.41 | 10.70 | 7.99 | 6.22 | 0.97 | 1.62 | 0.97 |
| | LPS | 48.08 | 4.84 | 24.01 | 3.40 | 88.46 | 69.96 | 12.96 | 0.58 |
| | CD40L | 77.61 | 13.39 | 37.00 | 8.75 | 126.88 | 12.70 | 10.71 | 4.50 |

These results demonstrate a high efficiency of transfection as well as inhibition of the NFκB signaling pathway comparable to the S0101627 compound.

### Example: Generation of Recombinant Adenovirus Capable of Expressing a Reporter or Regulatory Gene Under the Control of a Selective Inflammation-Related Cis-Element

This example illustrates the construction of recombinant adenovirus constructs to map signaling pathways related to inflammation. Subsequent examples will describe their use. In this example, the recombinant adenoviruses are constructed based on the BD ADENO-X polynucleotide, commercially available from BD Biosciences Clontech. The use of other recombinant adenoviruses is contemplated, and the use of ADENO-X should not be considered limiting. ADENO-X viral DNA is about 33 kb in length. It is derived from an adenovirus type 5 genome, and has been altered by deleting large portions of the adenovirus' E1 and E3 region. FIGURE 4 provides a schematic diagram of the recombinant adenovirus vector and the promoter/reporter system inserted therein.

As shown in FIGURE 4, the insert contains a transcription blocker upstream of a cis-acting enhancer element which is operationally linked to a luciferase reporter gene. The six cis-elements portrayed in FIGURE 4 are examples and are non-limiting. It is contemplated that other cis-elements may be used besides those listed. It is contemplated that other reporter genes may be used instead of luciferase. It is also contemplated that the insert contains a regulatory gene such as the IκBsd superdominant mtuant instead of a reporter gene.

As shown in FIGURE 4, the insert is cloned into the E1 region of the recombinant adenovirus. This recombinant adenovirus construct is rescued by transfecting it into HEK293 cells, which are then amplified to produce high titer adenovirus stocks. A virus titer of approximately 1x10⁹ pfu/ml in HEK293 is routinely achieved upon two cycles of amplification.

### Example: Infection of Primary Human Immunocytes by Reporter Recombinant Adenoviruses

In this example, recombinant adenoviruses capable of expressing a reporter gene under the control of specific cis-elements are used to map specific signaling pathways. Table 3 lists inflammation related cis-elements that are used to generate these recombinant adenoviruses, along with their respective transcription factors and signaling pathways.

**Table 3. Inflammation-Related Signaling Pathways**

| Cis-acting enhancer element | Abbr | Txn. factors | Signaling Pathways |
|---|---|---|---|
| Activator protein 1 | AP-1 | c-jun/c-fos | JNK pathway |
| CAMP response element | CRE | ATF2/CREB | JNK/p38 & PKA |
| IFN-stimulated response element | ISRE | STAT1/2 | Type I IFN |
| Nuclear factor, activated T cells | NFAT | NFAT | PKC & Ca⁺⁺ Calcineurin |
| Nuclear factor of kB cells | NFκB | NFkB | IKK/NFkB |
| Serum response element | SRE | Elk-1/SRF | MAPK/JNK |

In this example, the reporter recombinant adenoviruses of the previous example are used to infect primary human immunocytes. The infected cells are then stimulated and reporter activity in response to the stimulation is observed.

The stimulation applied to the infected cells depends on the type of cell infected. For example, CD4⁺ T cells are stimulated with either PMA/Ionomycin ("PT') or antibodies against CD3 and CD28 in the presence of protein G. Alternately, macrophages such as plate-attached macrophages derived from CD14⁺ monocytes are stimulated with lipopolysaccharide ("LPS"). Similarly, dendritic cells such as *in vitro* differentiated dendritic cells are stimulated with LPS.

After a certain amount of time post stimulation, the cells are collected and luciferase activity is measured. Luciferase activity can be measured by an enzyme assay using a photon generating substrate and detecting photons by luminometer.

In this example, CD4⁺ T cells are prepared as follows: First, peripheral blood mononuclear cells ("PBMCs") are obtained by density gradient centrifugation. One density gradient centrifugation medium is FICOLL PAQUE, commercially available from Amersham Biosciences. CD4⁺ T cells are then isolated from the PBMCs by magnetic depletion of CD8⁺ T cells, B cells, NK cells, and monocytes.

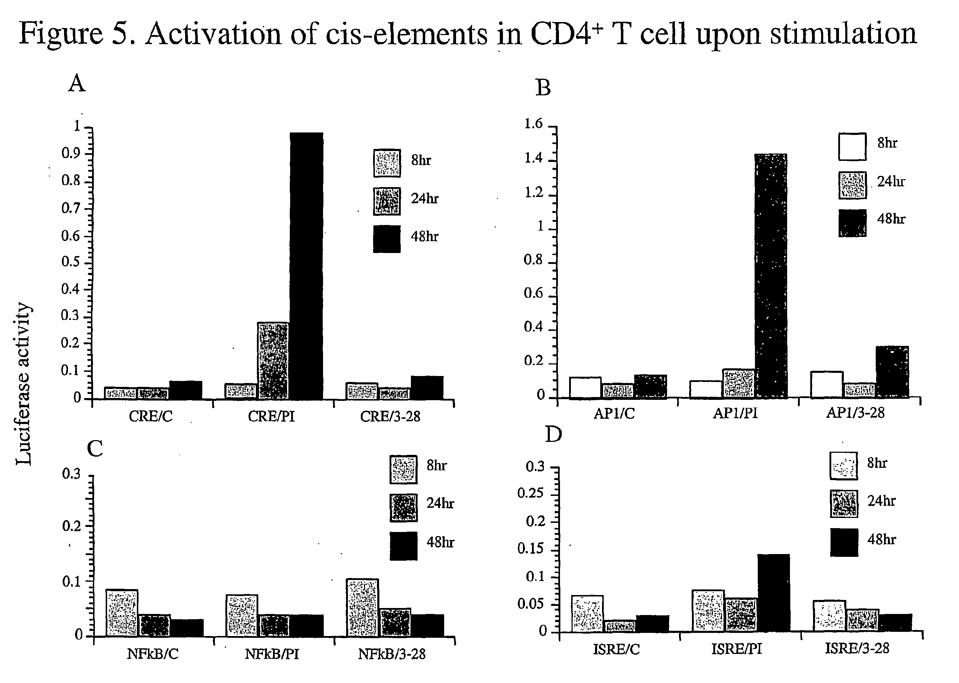

In this example, resting cultures of isolated CD4⁺ T cells are then infected with a recombinant adenovirus containing the luciferase gene which is transcribed under the control of a specific cis-element. In this example, four recombinant adenovirus constructs are used to infect four separate cultures of resting CD4⁺ T cells, each construct containing one of four cis-elements: CRE, AP-1, NOB, and ISRE. After 16 hours of incubation after infection, the cells are then either (1) mock stimulated ("C"), (2) stimulated with 20ng/ml PMA and 0.5ug/ml Ionomycin ("PI"), or (3) stimulated with 10 ng/ml anti-CD3 mAb, 5ug/ml anti-CD28 mAb, and 10ug/m Protein G ("3-28"). At 8 hours, 24 hours, and 48 hours post stimulation, the cells are then harvested and luciferase activity is measured. Luciferase activity can be measured by luminometer. FIGURE 5A is a chart depicting luciferase activity of CD4⁺ T cells infected with a recombinant adenovirus containing a CRE cis-element. FIGURE 5B is a chart depicting luciferase activity of CD4⁺ T cells infected with a recombinant adenovirus containing an AP-1 cis-element. FIGURE 5C is a chart depicting luciferase activity of CD4⁺ T cells infected with a recombinant adenovirus containing a NFκB cis-element. FIGURE 5D is a chart depicting luciferase activity of CD4⁺ T cells infected with a recombinant adenovirus containing a ISRE cis-element. These figures show significant activation of the CRE and AP-1 cis-elements 48 hours after PI stimulation, and no significant activation of any other combination of cis-element and stimulation.

Similar experiments were performed for CD14⁺ plate-bound macrophages. In this example, macrophages are obtained as follows: First, peripheral blood mononuclear cells ("PBMCs") are obtained by density gradient centrifugation. One density gradient centrifugation medium is FICOLL PAQUE, commercially available from Amersham Biosciences. Monocytes are then isolated from the PBMCs by magnetic depletion of non-monocytes. Finally, macrophages are isolated from the monocytes by attachment of the monocytes to a tissue culture plate in the absence of fetal bovine serum ("FBS").

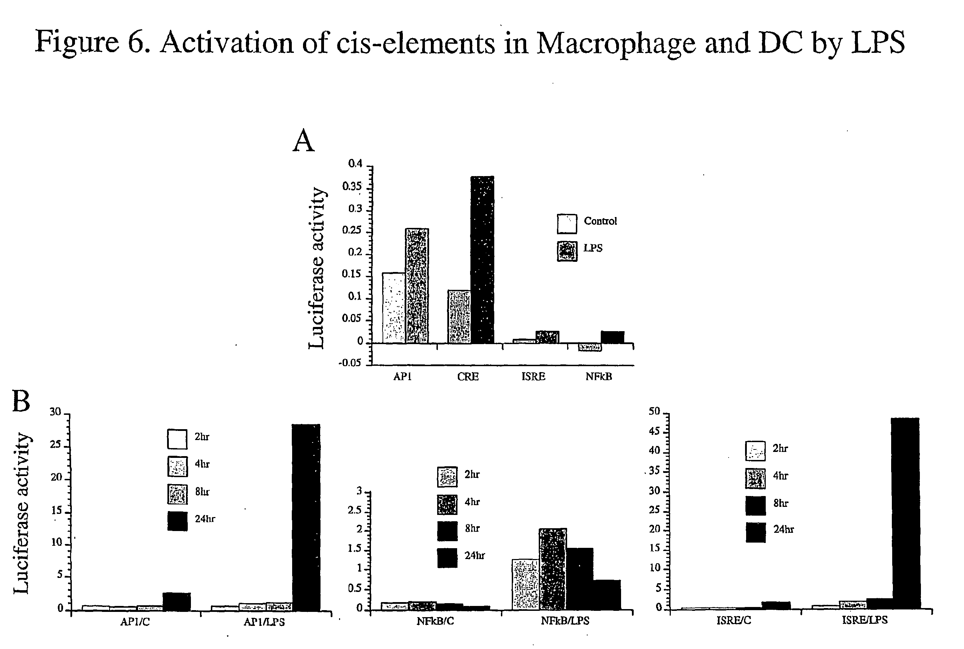

In this example, resting cultures of isolated macrophages are then infected with a recombinant adenovirus containing the luciferase gene which is transcribed under the control of a specific cis-element. In this example, four recombinant adenovirus constructs are used to infect four separate cultures of macrophages, each construct containing one of four cis-elements: CRE, AP-1, NFκB, and ISRE. The macrophages are infected at a MOI of 100 per cell. After 16 hours of incubation post infection, the macrophages are then either mock stimulated ("Control") or stimulated with lipopolysaccharide ("LPS") at the concentration of 1 µg/ml. At 24 hours post stimulation, cells are harvested and luciferase activity is measured by luminometer. FIGURE 6A is a chart depicting luciferase activity of four groups of macrophages each infected with one of four recombinant adenoviruses, each recombinant adenovirus containing an AP-1 cis-element, a CRE cis-element, an ISRE cis-element, and a NFκB cis-element, respectively. This figure demonstrates a significant base level activation, as well as significant stimulated activation, of the AP-1 and CRE cis-elements.

Similar experiments were performed for *in vitro* differentiated dendritic cells ("iDCs"). In this example, iDCs are obtained as follows: First, peripheral blood mononuclear cells ("PBMCs") are obtained by density gradient centrifugation. One density gradient centrifugation medium is FICOLL PAQUE, commercially available from Amersham Biosciences. Monocytes are then isolated from the PBMCs by magnetic depletion of non-monocytes. Finally, iDCs are isolated from the monocytes by treatment of the monocytes with IL4 and GM-GSF for 6 days.

In this example, resting cultures of isolated iDCs are then infected with a recombinant adenovirus containing the luciferase gene which is transcribed under the control of a specific cis-element. In this example, three recombinant adenovirus constructs are used to infect three separate cultures of iDCs, each construct containing one of three cis-elements: AP-1, NFκB, and ISRE. The iDCs are infected at a MOI of 100 per cell. After 16 hours of incubation post infection, the iDCs are then either mock stimulated ("C") or stimulated with lipopolysaccharide ("LPS") at the concentration of 1 µg/ml. At 2 hours, 4 hours, 8 hours, and 24 hours post stimulation, cells are harvested and luciferase activity is measured by luminometer. FIGURE 6B contains three charts depicting the time course of AP-1, NFκB, and ISRE stimulation, respectively. These charts demonstrate significant stimulation for all three cis-elements. These charts also indicate different time courses of activation.

### Example: Use of Adenovirus Reporter System for the Target Validation and In Vitro Compound Enabling in the Drug Discovery Process

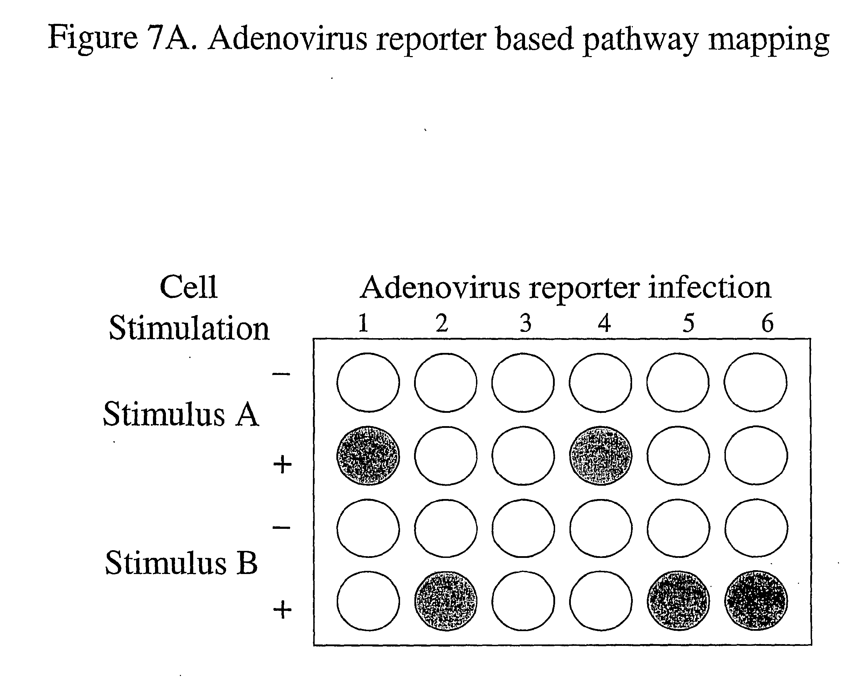

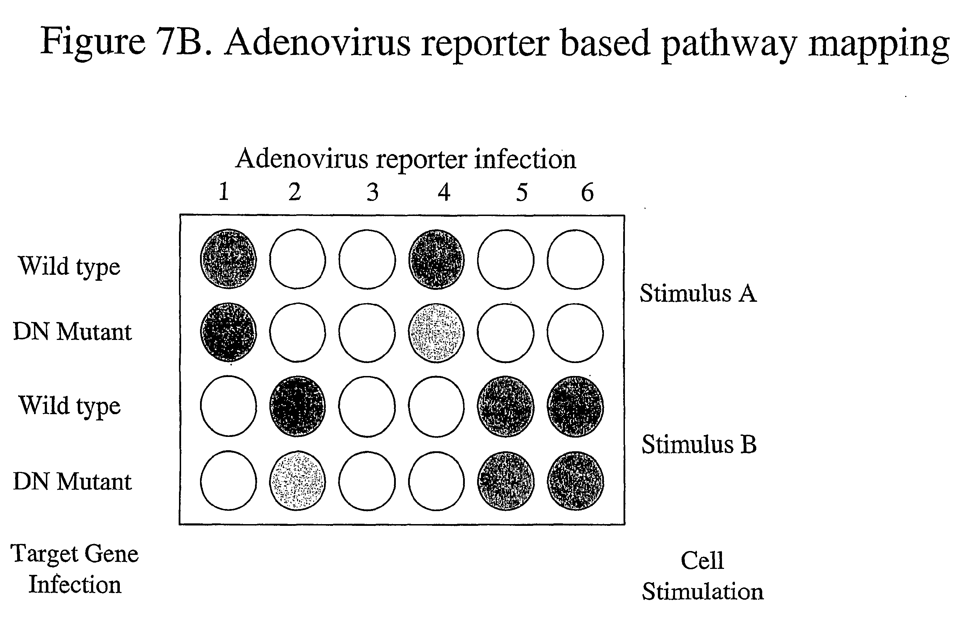

The previous examples demonstrate that selective activation as well as time course activation of transcription factors in primary human immunocytes upon specific stimulation. These observations enable the use of these adenovirus based transcription factor activation reporter systems for both target validation and compound enabling in the drug discovery process. FIGURES 7A and 7B are schematics depicting this process.

In this example, target cells are infected with different recombinant adenovirus reporters, depicted as columns 1 to 6 in FIGURES 7A and 7B. Then stimuli are added to the cultures. After incubation for specific time period, luciferase activities are measured. Filled circles represent hypothetical luciferase activity. No or undetectable luciferase activity may be registered without treatment with stimulus. If a stimulus activates the binding of transcription factor to its response element, for example, c-jun binds to AP-1 enhancer element, transcription would be induced and the luciferase activity can be detected. Different stimuli, represented by rows A and B in FIGURE 7A, may activate different signaling pathways.

Once this is established, co-infection of dominant negative mutant, such as IκBsd, of a target gene can be achieved in conjunction with reporter adenovirus. Activation of the specific signaling pathway the target is potentially involved in may decrease (and registered as reduced luciferase activity) in its activation upon specific stimulation. FIGURE 7B is a schematic depicting recombinant adenovirus reporter activity in the presence and absence of a co-infection of a dominant negative mutant.

## Claims

1. A method of determining whether a candidate cis-acting regulatory element belongs to a signaling pathway in an immunocyte, said method comprising the steps of:
a. transfecting said immunocyte with a recombinant adenovirus, said recombinant adenovirus comprising a reporter gene operatively linked to said candidate cis-acting regulatory element;
b. measuring a base level of reporter gene activity;
c. applying said stimulus to said immunocyte, said stimulus being known to modulate expression of said signaling pathway; and
d. measuring reporter gene activity in response to said stimulus.

2. The method of claim 1 wherein said stimulus comprises modulating expression of a regulatory protein and said applying step (c) comprises modulating the expression of said regulatory protein.

3. The method of claim 2 further comprising the step of co-transfecting said immunocyte with an expression system for said regulatory protein.

4. The method of claim 1 wherein said applying step (c) comprises introducing a candidate regulatory compound.

5. The method of claim 1 wherein said reporter gene is selected from the group consisting of: luciferase, green fluorescent protein ("GFP"), p-galactosidase ("GAL"), chloramphenicol acetyltransferase ("CAT").

6. The method of claim 1 wherein said reporter gene is a suppressor gene.

7. The method of claim 6 wherein said supressor gene is IKBsd.

8. The method of claim 1 wherein said cis-acting regulatory element is modulated by regulatory proteins related to inflammation.

9. The method of claim 1 wherein said cis-acting regulatory element is selected from the group consisting of: AP-1, CRE, ISRE, NFAT, NFKB, and SRE.

10. The method of claim 1 wherein said immunocyte is selected from the group consisting of: macrophage, CD4+ T cell, and immature dendritic cell.

## Patentansprüche

1. Verfahren zur Bestimmung davon, ob ein cis wirkendes regulatorisches Kandidatenelement zu einem Signalweg in einem Immunozyten gehört, wobei das Verfahren die folgenden Schritte umfasst:
a. Transfizieren des Immunozyten mit einem rekombinanten Adenovirus, wobei das rekombinante Adenovirus ein Reportergen in operativer Verknüpfung mit dem cis wirkenden regulatorischen Kandidatenelement umfasst;
b. Messen eines Grundniveaus an Reportergen-Aktivität;
c. Applizieren des Reizes an den Immunozyten, wobei der Reiz bekanntermaßen die Expression des Signalwegs moduliert; und
d. Messen von Reportergen-Aktivität als Antwort auf den Reiz.

2. Verfahren nach Anspruch 1, wobei der Reiz das Modulieren der Expression eines regulatorischen Proteins und der Applikationsschritt (c) das Modulieren der Expression des regulatorischen Proteins umfasst.

3. Verfahren nach Anspruch 2, ferner umfassend den Schritt des Cotransfizierens des Immunozyten mit einem Expressionssystem für das regulatorische Protein.

4. Verfahren nach Anspruch 1, wobei der Applikationsschritt (c) das Einführen einer regulatorischen Kandidatenverbindung umfasst.

5. Verfahren nach Anspruch 1, wobei das Reportergen ausgewählt ist aus der Gruppe: Luciferase, grünes Fluoreszenzprotein ("GFP"), p-Galactosidase ("GAL"), Chloramphenicol-Acetyltransferase ("CAT").

6. Verfahren nach Anspruch 1, wobei es sich bei dem Reportergen um ein Suppressorgen handelt.

7. Verfahren nach Anspruch 6, wobei es sich bei dem Reportergen um IKBsd handelt.

8. Verfahren nach Anspruch 1, wobei das cis wirkende regulatorische Element durch in Verbindung mit Entzündung stehende regulatorische Proteine moduliert wird.

9. Verfahren nach Anspruch 1, wobei das cis wirkende regulatorische Element ausgewählt ist aus der Gruppe: AP-1, CRE, ISRE, NFAT, NFKB und SRE.

10. Verfahren nach Anspruch 1, wobei der Immunozyt ausgewählt ist aus der Gruppe: Makrophage, CD4+-T-Zelle und unreife dendritische Zelle.

## Revendications

1. Méthode pour déterminer si un élément régulateur candidat agissant en cis appartient à une voie de signalisation dans un immunocyte, ladite méthode comprenant les étapes consistant à :
a. transfecter ledit immunocyte avec un adénovirus recombinant, ledit adénovirus recombinant comprenant un gène rapporteur lié de façon opérationnelle audit élément régulateur candidat agissant en cis ;
b. mesurer un taux basal d'activité du gène rapporteur ;
c. appliquer ledit stimulus audit immunocyte, ledit stimulus étant connu pour moduler l'expression de ladite voie de signalisation ; et
d. mesurer l'activité du gène rapporteur en réaction audit stimulus.

2. Méthode selon la revendication 1 **caractérisée en ce que** ledit stimulus comprend la modulation de l'expression d'une protéine de régulation et ladite étape (c) d'application comprend la modulation de l'expression de ladite protéine de régulation.

3. Méthode selon la revendication 2 comprenant en outre l'étape de co-transfection dudit immunocyte avec un système d'expression pour ladite protéine de régulation.

4. Méthode selon la revendication 1, **caractérisée en ce que** ladite étape (c) d'application comprend l'introduction d'un composé régulateur candidat.

5. Méthode selon la revendication 1, **caractérisée en ce que** ledit gène rapporteur est choisi dans le groupe constitué de : luciférase, protéine fluorescente verte ("GFP"), p-galactosidase ("GAL"), chloramphénicol acétyltransférase ("CAT").

6. Méthode selon la revendication 1, **caractérisée en ce que** ledit gène rapporteur est un gène suppresseur.

7. Méthode selon la revendication 6, **caractérisée en ce que** ledit gène suppresseur est IKBsd.

8. Méthode selon la revendication 1, **caractérisée en ce que** ledit élément régulateur agissant en cis est modulé par des protéines de régulation associées à l'inflammation.

9. Méthode selon la revendication 1, **caractérisée en ce que** ledit élément régulateur agissant en cis est choisi dans le groupe constitué de : AP-1, CRE, ISRE, NFAT, NFKB et SRE.

10. Méthode selon la revendication 1, **caractérisée en ce que** ledit immunocyte est choisi dans le groupe constitué de : macrophage, cellule T CD4+, et cellule dendritique immature.
